# NOUVEAU FASCICULE DE BREVET EUROPEEN

(11) **EP 0 681 829 B2**
(45) Date de publication et mention de la décision concernant l'opposition: **03.08.2011**
(45) Mention de la délivrance du brevet: 09.10.1996
(21) Numéro de dépôt: 95400532.8
(22) Date de dépôt: 13.03.1995
(51) Int. Cl.: A61Q 5/10

(54) **Composition de teinture d'oxydation des fibres kératiniques**
Oxidationsfärbemittel für Keratinfasern
Oxidative dyeing composition for keratinous fibres

(30) Priorité: 09.05.1994 FR 9405688
(43) Date de publication de la demande: 15.11.1995
(73) Titulaire: L'Oréal, 75008 Paris (FR)
(72) Inventeur: Bone, Eric, F-92110 Clichy (FR); De la Mettrie, Roland, F-78110 Le Vesinet (FR)
(74) Mandataire: Dossmann, Gérard

(56) Documents cités:
- EP-A- 0 039 806
- EP-A- 0 358 550
- EP-A- 0 360 644
- EP-A- 0 634 164
- EP-A1- 0 459 900
- DE-C- 4 301 663
- FR-A- 2 156 527
- GB-A- 2 018 836

## Description

La présente invention a pour objet une composition pour la teinture d'oxydation des fibres kératiniques et en particulier des fibres kératiniques humaines telles que les cheveux, mettant en oeuvre un précurseur de colorant d'oxydation en association avec un coupleur et un agent oxydant, en milieu acide ainsi que le procédé de teinture mettant en oeuvre cette composition.

Cette composition est particulièrement destinée à la teinture des cheveux gris ou blancs qui ont tendance à jaunir.

Il est connu de teindre les fibres kératiniques et en particulier les cheveux humains avec des compositions tinctoriales contenant des précurseurs de colorant d'oxydation, en particulier des ortho ou paraphénylénediamines, des ortho ou paraaminophénols appelés généralement bases d'oxydation. Les précurseurs de colorants d'oxydation, ou bases d'oxydation, sont des composés incolores ou faiblement colorés qui une fois mélangés à des produits oxydants, au moment de l'emploi, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, choisis notamment parmi les métadiamines aromatiques, les métaaminophénols et les metadiphènols.

Dans le cas particulier de la coloration des cheveux gris ou blancs et plus particulièrement pour remédier à leur jaunissement, tout en leur laissant un léger reflet argenté ou acier, il est possible d'employer plusieurs type de produits.

Il existe tout d'abord des shampooings à base de colorants directs présents en faible quantité. Les colorants directs sont mis en oeuvre sans agent oxydant et sont capables d'apporter à la coloration naturelle des cheveux, une modification plus ou moins marquée. Cependant ces shampooings, bien que donnant un effet immédiat, ont l'inconvénient de donner un résultat disparaissant entre deux shampooings.

Il existe également des produits colorants présentés sous forme de mousse ou de solution contenant des colorants directs qui sont appliqués pendant des temps de pause courts sur les cheveux. Cependant de tels produits confèrent aux cheveux des teintures sélectives, c'est à dire donnant des nuances différentes sur cheveux sensibilisés ou non sensibilisés. De plus, ces teintures ne couvrent pas assez les cheveux et disparaissent rapidement après quelques shampooings.

Enfin, dans le domaine de la coloration d'oxydation classique, la formulation de véritables nuances grises est très limitée et est réalisée en mettant en oeuvre des procédés de teinture présentant des temps de pause relativement longs entraînant une sélectivité importante et un résultat tinctorial manquant de naturel. De plus, les procédés de teinture d'oxydation sont généralement réalisés à pH alcalin en mettant en oeuvre des agents alcalinisants puissants tel que l'ammoniaque, ce qui a pour effet de détériorer les fibres kératiniques.

C'est pour résoudre ces différents problèmes que la demanderesse a mis au point la composition tinctoriale de l'invention.

Elle vient de découvrir que l'utilisation de certains précurseurs de colorant d'oxydation en association avec certains coupleurs et un agent oxydant, en milieu acide, permettait d'obtenir une composition tinctoriale permettant de remédier à ces problèmes et conduisant, après un temps de pause court, à des nuances grises, naturelles, peu sélectives, couvrantes et remédiant au jaunissement des cheveux gris et de plus tenaces vis à vis de l'action de la transpiration, des shampooings, des traitements chimiques ou des agents atmosphériques tels que la lumière.

La présente invention a donc pour objet une composition pour la teinture d'oxydation des fibres kératiniques, en particulier des fibres kératiniques humaines telles que les cheveux, caractérisée par le fait qu'elle résulte du mélange extemporané d'une part, d'une composition (A) contenant, dans un milieu approprié pour la teinture :
- au moins un précurseur de colorant d'oxydation encore appelé "base d'oxydation" de formule (I) : 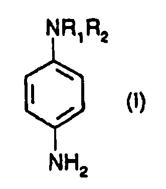 dans laquelle :
   - R₁ représente un atome d'hydrogène ou un radical mono- ou polyhydroxyalkyle en C₂-C₆ ;
   - R₂ représente un radical mono- ou polyhydroxyalkyle C₂-C₈; β-aminoéthyle ou bien un groupement de formule (II) suivante: 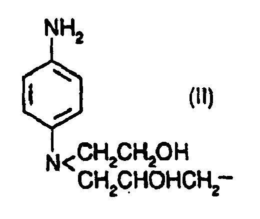 ou au moins l'un de ses sels cosmétiquement acceptable,
- et au moins un coupleur choisi parmi le méta-aminaphénol, le 2-méthyl 5-amoino phénol, le méta-diphenol le 2-méthyl 1,3-dihydroxybenzène et les sels cosmétiquement acceptables de ces composés,
et d'autre part, d'une composition (B) contenant, dans un milieu approprié pour la teinture, au moins un agent oxydant, le pH des compositions (A) et (B) étant tel qu'après mélange de la composition (A) avec la composition (B) dans un rapport pondéral variant de préférence de 0.5 à 5 et encore plus préférentiellement de 1 à 3, le pH de la composition résultante soit inférieur à 7, le pH de la composition résultant du mélange extemporané des compositions (A) et (B) ayant une valeur inférieure à 7.

L'invention a également pour objet un procédé de teinture d'oxydation des fibres kératiniques mettant en oeuvre cette composition.

Dans les compositions de l'invention, les sels des composés de formule (I) et des coupleurs sont notamment choisis parmi les chlorhydrates, les bromhydrates, les sulfates et les tartrates.

Parmi les radicaux mono- polyhydroxyalkyle C₂-C₆ définis pour R₁ et R₂, en préfère les radicaux β-hydroxyéthyle et β,γ-dihydroxypropyle.

Parmi les composés de formule (I), on peut citer :
- la N,N-bis-(β-hydroxyéthyl)amino paraphénylènediamine,
- le 1-amino 4-(N-β-hydroxyéthyl, N-β-aminoéthyl)amino benzène,
- le N,N'-bis-(β-hydroxyéthyl) N,N'-bis-(4'-aminophényl) 1,3-diamino 2-propanol,
ainsi que leurs sels cosmétiquement acceptables.

L'agent oxydant est choisi parmi les agents oxydants classiquement utilisés en coloration d'oxydation et de préférence parmi le peroxyde d'hydrogéne, le peroxyde d'urée, les bromates de métaux alcalins, les persels tels que les perborates et persulfates. Le peroxyde d'hydrogène est particulièrement préféré.

Le pH de la composition résultant du mélange extemporané des composants (A) et (B) a une valeur inférieure à 7 et est compris de préférence entre 3 et 7.

Le pH de la composition (A) telle que définie ci-dessus peut varier entre 3 et 11 et peut être ajusté à la valeur désirée au moyen d'agents alcalinisants habituellement utilisés en teinture des fibres kératiniques, tels que l'ammoniaque, les carbonates alcalins, les alcanolamines telles que les mono-, di- et triéthanolamines ainsi que leurs dérivés ou au moyen d'agents acidifiants classiques tels que les acides minéraux ou organiques comme l'acide chlorhydriques l'acide orthophosphorique, les acides carboxyliques comme l'acide tartrique, l'acide citrique, l'acide lactique, les acides sulfoniques.

Le pH de la composition (B) telle que définie ci-dessous est de préférence intérieur à 7. Ce pH peut avoir une valeur minimum de 1 et de preférence inférieure à 5. Ce composant peut être acidifié avec le même type d'agents acidifiants que ceux utilisés pour la composition (A).

Le précurseur d'oxydation de formule (I) est présent en des proportions de préférence comprises entre 0,01% et 4% en poids environ par rapport au poids total de la composition résultante et encore plus préférentiellement entre 0,1 % et 2% en poids,

Les coupleurs de l'invention, c'est à dire le mèta-aminophénol, le 2-méthyl 5-amino phénol, le méta-diphénol, le 2-méthyl 1,3-dihydroxybenzène ou leurs sels sont présents en des proportions de préférence comprises entre 0.005 % et 5 % en poids environ par rapport au poids total de la composition résultante et encore plus préférentiellement entre 0,01% et 3,5% en poids.

La composition (A) définie ci-dessus peut également contenir à titre secondaire, en plus des précurseurs de colorants de formule (I), d'autres précurseurs de colorants d'oxydation, de type para ou ortho et/ou d'autres coupleurs différents de ceux définis précédemment et /ou des colorants directs de façon à obtenir des nuances de teintes particulières.

Le milieu approprié pour la teinture est généralement aqueux mais il peut également contenir des solvants organiques pour solubiliser les composés qui ne seraient pas suffisamment solubles dans l'eau. Parmi ces solvants, on peut citer à titre d'exemple, les alcanois inférieurs en C₁-C₄. tels que l'éthanol et l'isopropanol; le glycérol; les glycols et éthers de glycols comme le 2-butoxyéthanol, le propylèneglycol, le monoéthyléther et le monométhyléther du diéthylèneglycol, ainsi que les alcools aromatiques comme l'alcool benzifique ou le phenoxyéthanol, les produits analogues et leurs mélanges.

Les solvants peuvent être présents dans des proportions de préférence comprises entre 1 et 40 % en poids par rapport au poids total de la composition tinctoriale et encore plus préférentiellement entre 5 et 30 % en poids.

Les compositions appliquées sur les cheveux peuvent également renfermer divers adjuvants utlisés classiquement dans les compositions pour la teinture des cheveux tels que des agents tensio-actits anioniques, cationiques, nonioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement, des agents filmogènes, des agents conservateurs, des agents opacifiants.

La composition appliquée sur les cheveux peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture d'oxydation des fibres kératiniques et en particulier des fibres kératiniques humaines telles que les cheveux mettant en oeuvre la composition telle que définie ci-dessus.

Selon ce procédé, on mélange au moment de l'emploi la composition (A) avec la composition (B), le rapport pondéral de la composition (A) à la composition (B) variant de préférence de 0,5 à 5, puis on applique la composition résultante sur les cheveux pendant un temps de pause court de préférence inférieur à 10 minutes et encore plus préférentiellement pendant un temps de pause compris entre 3 et 5 minutes environ. Les cheveux sont ensuite rincés.

Le rapport pondéral de la composition (A) à la composition (B) varie encore plus préférentiellement de 1 à 3.

Les exemples qui suivent sont destinés à illustrer l'invention sans pour autant en limiter la portée.

### EXEMPLES DE COMPOSITIONS TINCTORIALES

### EXEMPLES 1 à 4

On prépare la composition (A) suivante :

| | |
|---|---|
| -Alcool oléique polyglycérolé à 2 moles de glycérol | 4,0 g |
| -Alcool oléique polyglycérolé à 4 moles de glycérol à 78 % de MA | 5,7 g MA |
| - Acide oléique | 3,0 g |
| - Amine oléique oxyéthylénée à 2 moles d'oxyde d'éthylène, vendue sous la dénomination ETHOMEEN 012 par la Société AKZO | 7,0 g |
| - Laurylaminosuccinamate de diéthylaminopropyle, sel de sodium à 55 % de MA | 3,0 g MA |
| - Alcool oléique | 5.0 g |
| - Diéthanolamine d'acide oléique | 12,0 g |
| - Propylèneglycol | 3,5 g |
| - Alcool éthylique | 7,0 g |
| -Dipropylèneglycol | 0,5 g |
| - Monométhyléther de propylèneglycol | 9,0 g |
| - Métabisulfite de sodium en solution aqueuse 35% de MA | 0,46 g MA |
| - Acétate d'ammonium | 0.8 g |
| - Antioxydant, séquestrant qs | |
| - Parfum, conservateur qs | |
| - Acide citrique | 0,12 g |
| - Précurseur de formule (I) | X |
| - Coupleur de l'invention | Y g |
| - Autres colorants | Z g |
| - Eau déminéralisée qsp | 100 g |

Au moment de l'emploi, on mélange cette composition (A) poids pour poids avec une composition (B) constituée par de l'eau oxygénée à 20 volumes (6 % en poids), et dont le pH est de 3.

On obtient une composition résultante dont le pH est indiqué dans le tableau ci-après.

On applique cette composition résultante sur des cheveux gris naturels à 90 % de blancs pendant 5 minutes. Après rinçage, lavage au shampooing, rinçage et séchage, les cheveux sont teints dans les nuances indiquées dans le tableau ci-après :

| **Exemples en g** | **1** | **2** | **3** | **4** |
|---|---|---|---|---|
| N,N'-bis-(β-hydroxyéthyl) N,N'-bis-(4'-aminophényl) 1,3-diamino 2-propanol, tétrachlorhodrate (précurseur de formule (I)) | 0,506 | 0,253 | - | - |
| N,N-bis-(β-hydroxyéthyl)amino paraphénylènediamine, sulfate (précurseur de formule (I)) | - | 0,158 | - | 0,312 |
| 1-amino 4-(N-β-hydroxyéthyl, N-β-aminoéthyl) amino benzène, dichlorhydrate (précurseur de formule (I)) | - | - | 0,40 | - |
| méta-diphénol (coupleur de l'invention) | - | - | 0,03 | 0,07 |
| méta-aminophénol (coupleur de l'invention) | 0,11 | - | - | - |
| 2-méthyl 1,3-dihydroxybenzéne (coupleur de l'invention) | - | 0,041 | - | 0,4 |
| 1-β-hydroxyéthyloxy 2,4-diaminobenzéne, dichlorhydrate (coupleur secondaire) | - | - | 0,03 | - |
| 2-méthyl 5-amino phénol (coupleur de l'invention) | - | 0,08 | 0,16 | - |
| pH de la composition résultante | 6,4 | 6,4 | 6,5 | 6,6 |
| Nuance obtenue | gris naturel | gris nacré | gris argent | gris léger nacré |

## Revendications

1. Composition pour la teinture d'oxydation des fibres kératiniques, en particulier des fibres kératiniques humaines telles que les cheveux **caractérisée par le fait qu'**elle résulte du mélange extemporané d'une part, d'une composition (A) contenant, dans un milieu approprié pour la teinture :
- au moins un précurseur de colorant d'oxydation encore appelé baste d'oxydation" de formule (I) : 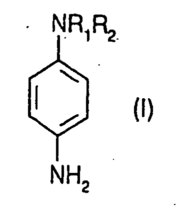 dans laquelle :
- R₁ représente un atome d'hydrogène ou un radical mono- ou polyhydroxyalkyle en C₂-C₆ ;
- R₂ représente un radical mono- ou polyhydroxyalkyle en C₂-C₆ ; β-aminoéthyle ou bien un groupement de formule (II) suivante : 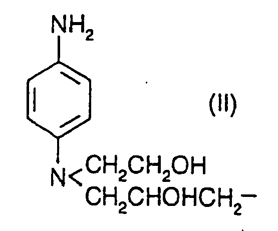 ou au moins l'un de ses sels cosmétiquement acceptable,
- et au moins un coupleur choisi parmi le méta-aminophénol, le 2-méthyl 5-amino phénol, le méta-diphénol le 2-méthyl 1,3-dihydroxybenzène et les sels cosmétiquement acceptables de ces composés, et d'autre part, d'une composition (B) contenant, dans un milieu approprié pour la teinture, au moins un agent oxydant,
le pH des compositions (A) et (B) étant tel qu'après mélange de la composition (A) avec la composition (B) dans un rapport pondéral variant de 0,5 à 5, le pH de la composition résultante soit inférieur à 7, le pH de la composition résultant du mélange extemporané des compositions (A) et (B) ayant une valeur inferieur à 7.

2. Composition selon la de revendication 1 **caractérisée par le fait que** les sels des composés de formule (I) et des coupleurs sont choisis parmi les chlorhydrates, les bromhydrates, les sulfates et les tartrates.

3. Composition selon l'une quelconque des revendications 1 ou 2 **caractérisé par le fait que** le radical mono- ou polyhydroxyalkyle en C₂-C₆ défini pour R₁ est un radical β-hydroxyéthyle ou β,γ-dihydroxypropyle.

4. Composition selon l'une quelconque des revendications 1 à 3 **caractérisée par le fait que** les composés de formule (I) sont choisis parmi la N,N-bis-(β-hydroxyéthyl)amino paraphénylènediamine, le 1-amino 4-(N-β-hydroxyéthyl, N-β-aminoéthyl)amino benzène, le N,N'-bis-(β-hydroxyéthyl) N,N'-bis-(4'-aminophényl) 1,3-diamino 2-propanol, ainsi que leurs sels cosmétiquement acceptables.

5. Composition selon l'une quelconque des revendications 1 à 4 **caractérisée par le fait que** l'agent oxydant est choisi parmi le peroxyde d'hydrogène, le peroxyde d'urée, les bromates de métaux alcalins et les persels tels que les perborates et persulfates.

6. Composition selon l'une quelconque des revendications 1 à 4 **caractérisée par le fait qu'**elle présente un pH compris entre 3 et inférieur à 7.

7. Composition selon l'une quelconque des revendications 1 à 6 **caractérisée par le fait que** le pH de la composition (A) est compris entre 3 et 11.

8. Composition selon l'une quelconque des revendications 1 à 7 **caractérisée par le fait que** le pH de la composition (B) a une valeur inférieure à 7.

9. Composition selon l'une quelconque des revendications 1 à 8 **caractérisée par le fait que** le précurseur d'oxydation de formule (I) est présent en des proportions de préférence comprises entre 0,01 % et 4 % en poids par rapport au poids total de la composition résultante.

10. Composition selon l'une quelconque des revendications 1 à 9 **caractérisée par le fait que** le méta-aminophénol et/ou le 2-méthyl 5-amino-phénol et/ou le métadiphériol et/ou le 2-méthyl 1,3-dihydroxybenzène et/ ou leurs sels sont présents en des proportions comprises entre 0,005 % et 5 % en poids par rapport au poids total de la composition résultante.

11. Composition selon l'une quelconque des revendications 1 à 10 **caractérisée par le fait que** la composition (A) contient, à titre secondaire, en plus des précurseurs de colorants de formule (I), d'autres précurseurs de colorants d'oxydation, de type para ou ortho et/ ou d'autres coupleurs différents de ceux définis dans la revendication 1 et/ou des colorants directs.

12. Composition selon l'une quelconque des revendications 1 à 11 **caractérisée par le fait que** le milieu approprié pour la teinture est constitué par de l'eau ou un mélange d'eau et d'un solvant choisi parmi les alcanols inférieurs en C₁-C₄, le glycérol; les glycols, les éthers de glycols, les alcools aromatiques ou leurs mélanges.

13. Composition selon l'une quelconque des revendications 1 à 12 **caractérisée par le fait qu'**elle contient en outre des agents tensio-actifs anioniques, cationiques, nonioniques, amphotères, zwittérioniques ou leurs mélanges, des agents épaississants, des agents anti-oxydants et 1 ou tout autre adjuvant cosmétiquement acceptable.

14. Procédé de teinture d'oxydation des fibres kératiniques et en particulier des fibres kératiniques humaines telles que les cheveux **caractérisé par le fait que** l'on appliqué sur ces fibres une composition résultant du mélange extemporané
d'une part, d'une composition (A) contenant, dans un milieu approprié pour la teinture:
- au moins un précurseur de colorant d'oxydation de formule (I) : 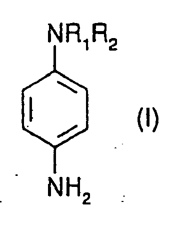 dans laquelle :
- R₁ représente un atome d'hydrogène ou un radical mono- ou polyhydroxyalkyle en C₂-C₆ ;
- R₂ représente un radical mono- ou polyhydroxyalkyle en C₂-C₆ ; β-aminoéthyle ou bien un groupement de formule (II) suivante : 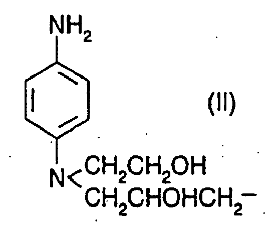 ou au moins l'un de ses sels cosmétiquement acceptable,
- et au moins un coupleur choisi parmi le méta-aminaphénol, le 2-méthyl 5-amino phénol, le méta-diphénol, le 2-méthyt 1,3-dihydroxybenzène et les sels cosmétiquement acceptables de ces composés,
et d'autre part, d'une composition (B) contenant, dans un milieu approprié pour la teinture; au moins un agent oxydant,
le pH des compositions (A) et (B) étant tel qu'après mélange de la composition (A) avec la composition (B) dans un rapport pondéral variant de 0,5 à 5, le pH de la composition résultante soit inférieur à 7, le pH de la composition résultant du mélange extemporané des compositions (A) et (B) ayant une valeur inférieure à 7.

15. Procédé selon la revendication 14 **caractérisé par le fait que** les composés de formule (I) sont choisis parmi la N,N-bis-(β-hydroxyéthyl)amino paraphénylènediamine, le 1-amino 4-(N-β-hydroxyéthyl, N-β-aminoéthyl)amino benzène, le N,N'-bis-β-hydroxyéthyl) N,N'-bis-(4'-aminophényl) 1,3-diamino 2-propanol, ainsi que leurs sels cosmétiquement acceptables.

16. Procédé selon la revendication 14 ou 15 **caractérisé par le fait que** l'agent oxydant est choisi parmi le peroxyde d'hydrogène, le peroxyde d'urée, les bromates de métaux alcalins et les persels tels que les perborates et persulfates.

17. Procédé selon l'une quelconque des revendications 14 à 16 **caractérisé par le fait que** le pH de la composition appliquée sur les fibres est compris entre 3 et inférieur à 7.

18. Procédé selon l'une quelconque des revendications 14 à 17 **caractérisé par le fait que** la composition est appliquée sur les fibres avec un temps de pause inférieur à 10 minutes.

19. Procédé selon l'une quelconque des revendications 14 à 18 **caractérisé par le fait que** la composition est appliquée sur les fibres avec un temps de pause compris entre 3 et 5 minutes.

## Claims

1. Composition for the oxidation dyeing of keratinous fibres, especially human keratinous fibres such as hair, **characterized in that** it results from the mixing, at the time of use,
on the one hand, of a composition (A) containing, in a medium appropriate for dyeing:
- at least one oxidation dye precursor, also called "oxidation base", of formula (I): 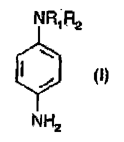 in which:
- R₁ represents a hydrogen atom or a C₂-C₆ mono- or polyhydroxyalkyl radical;
- R₂ represents a C₂-C₆ mono- or polyhydroxyalkyl radical, β-aminoethyl or a group of the following formula (II): 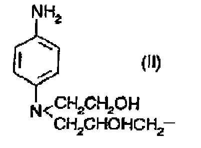 or at least one of its cosmetically acceptable salts,
- and at least one coupler chosen from meta-aminophenol, 2-methyl-5-aminophenol, meta-diphenol, 2-methyl-1,3-dihydroxybenzene and the cosmetically acceptable salts of these compounds,
and, on the other hand, of a composition (B) containing, in a medium appropriate for dyeing, at least one oxidizing agent,
the pH of the compositions (A) and (B) being such that, after mixing composition (A) with composition (B) in a ratio by weight which ranges from 0.5 to 5, the pH of the resulting composition is less than 7, the pH of the composition resulting from the mixing, at the time of use, of compositions (A) and (B) having a value of less than 7.

2. Composition according to Claim 1, **characterized in that** the salts of the compounds of formula (I) and of the couplers are chosen from hydrochlorides, hydrobromides, sulphates and tartrates.

3. Composition according to either of Claims 1 and 2, **characterized in that** the C₂-C₆ mono- or polyhydroxyalkyl radical defined for R₁ is a β-hydroxyethyl or β, γ-dihydroxypropyl radical.

4. Composition according to any one of Claims 1 to 3, **characterized in that** the compounds of formula (I) are chosen from N,N-bis(β-hydroxyethyl)amino-paraphenylenediamine, 1-amino-4-(N-β-hydroxyethyl-N-β-aminoethyl)aminobenzene, N,N'-bis(β-hydroxyethyl)-N,N'-bis(4'-aminophenyl)-1,3-diamino-2-propanol and the cosmetically acceptable salts thereof.

5. Composition according to any one of Claims 1 to 4, **characterized in that** the oxidizing agent is chosen from hydrogen peroxide, urea peroxide, alkali metal bromates and persalts such as perborates and persulphates.

6. Composition according to any one of Claims 1 to 4, **characterized in that** it has a pH of between 3 and less than 7.

7. Composition according to any one of Claims 1 to 6, **characterized in that** the pH of composition (A) is between 3 and 11.

8. Composition according to any one of Claims 1 to 7, **characterized in that** the pH of composition (B) has a value of less than 7.

9. Composition according to any one of Claims 1 to 8, **characterized in that** the oxidation precursor of formula (I) is present in proportions which are preferably between 0.01 % and 4 % by weight relative to the total weight of the resulting composition.

10. Composition according to any one of Claims 1 to 9, **characterized in that** meta-aminophenol and/or 2-methyl-5-aminophenol and/or meta-diphenol and/or 2-methyl-1,3-dihydroxybenzene and/or their salts are present in proportions of between 0.005 % and 5 % by weight relative to the total weight of the resulting composition.

11. Composition according to any one of Claims 1 to 10, **characterized in that** the composition (A) contains, secondarily and in addition to the dye precursors of formula (I), other oxidation dye precursors of the para or ortho type and/or other couplers which are different from those defined in Claim 1 and/or direct dyes.

12. Composition according to any one of Claims 1 to 11, **characterized in that** the medium appropriate for dyeing consists of water or a mixture of water and a solvent chosen from C₁-C₄ lower alkanols, glycerol, glycols, glycol ethers, aromatic alcohols or mixtures thereof.

13. Composition according to any one of Claims 1 to 12, **characterized in that** it additionally contains anionic, cationic, nonionic, amphoteric or zwitterionic surfactants or mixtures thereof, thickeners, antioxidants and/or any other cosmetically acceptable adjuvant.

14. Process for the oxidation dyeing of keratinous fibres, and especially human keratinous fibres such as hair, **characterized in that** a composition is applied to
these fibres which results from the mixing, at the time of use,
on the one hand, of a composition (A) containing, in a medium appropriate for dyeing:
- at least one oxidation dye precursor of formula (I): 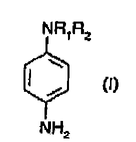 in which:
- R₁ represents a hydrogen atom or a C₂-C₆ mono- or polyhydroxyalkyl radical;
- R₂ represents a C₂-C₆ mono- or polyhydroxyalkyl radical, β-aminoethyl or a group of the following formula (II): 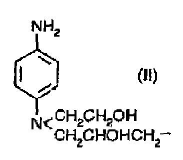 or at least one of its cosmetically acceptable salts,
- and at least one coupler chosen from meta-aminophenol, 2-methyl-5-aminophenol, meta-diphenol, 2-methyl-1,3-dihydroxybenzene and the cosmetically acceptable salts of these compounds,
and, on the other hand, of a composition (B) containing, in a medium appropriate for dyeing, at least one oxidizing agent,
the pH of the compositions (A) and (B) being such that, after mixing composition (A) with composition (B) in a ratio by weight which ranges from 0.5 to 5, the pH of the resulting composition is less than 7, the pH of the composition resulting from the mixing, at the time of use, of compositions (A) and (B) having a value of less than 7.

15. Process according to Claim 14, **characterized in that** the compounds of formula (I) are chosen from N,N-bis(β-hydroxyethyl)amino-para-phenylenediamine, 1-amino-4-(N-β-hydroxyethyl-N-β-aminoethyl)aminobenzene, N,N'-bis(β-hydroxyethyl)-N,N'-bis(4'-aminophenyl)-1,3-diamino-2-propanol and the cosmetically acceptable salts thereof.

16. Process according to Claim 14 or 15, **characterized in that** the oxidizing agent is chosen from hydrogen peroxide, urea peroxide, alkali metal bromates and persalts such as perborates and persulphates.

17. Process according to any one of Claims 14 to 16, **characterized in that** the pH of the composition applied to the fibres is between 3 and less than 7.

18. Process according to any one of Claims 14 to 17, **characterized in that** the composition is applied to the fibres with a waiting time of less than 10 minutes.

19. Process according to any one of Claims 14 to 18, **characterized in that** the composition is applied to the fibres with a waiting time of between 3 and 5 minutes.

## Patentansprüche

1. Zusammensetzung zum oxidativen Färben von Keratinfasern und insbesondere von menschlichen Keratinfasern, wie Haaren, **dadurch gekennzeichnet, dass** sie durch Mischen unmittelbar vor der Verwendung einerseits einer Zusammensetzung (A), die in einem zum Färben geeigneten Medium enthalt:
· mindestens ein Farbstoffvorprodukt eines Oxidationsfarbstoffes, das auch "Oxidationsbase" genannt wird, der Formel (I): 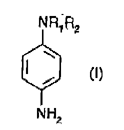 worin bedeuten:
R₁ Wasserstoff oder eine Mono- oder Polyhydroxyalkylgruppe mit 2 bis 6 Kohlenstoffatomen,
R₂ eine Mono- oder Polyhydroxyalkylgruppe mit 2 bis 6 Kohlenstoffatomen, β-aminomethyl oder auch eine Gruppe der folgenden Formel (II): 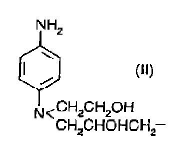
oder mindestens ein kosmetisch akzeptables Salz dieser Verbindungen,
· und mindestens einen Kuppler, der unter m-Aminophenol, 2-Methyl-5-aminophenol, m-Dihydroxybenzol, 2-Methyl-1,3-dihydroxybenzol und den kosmetisch akzeptablen Salzen dieser Verbindungen ausgewählt ist,
und andererseits einer Zusammensetzung (B) resultiert, die in einem zum Färben geeigneten Medium mindestens ein Oxidationsmittel enthält,
wobei der pH-Wert der Zusammensetzungen (A) und (B) so ist, dass nach dem Mischen der Zusammensetzung (A) mit der Zusammensetzung (B) in einem Gewichtsverhältnis von 0,5 bis 5 der pH-Wert der resultierenden Zusammensetzung unter 7 liegt, wobei der pH-Wert des bedarfsgemäß gebildeten Gemisches der Zusammensetzung (A) und (B) einen Wert unter 7 aufweist.

2. Zusammensetzung nach Anspruch 1, **dadurch gekennzeichnet, dass** die Salze der Verbindungen der Formel (I) und der Kuppler unter Hydrochloriden, Hydrobromiden, Sulfaten und Tartraten ausgewählt sind.

3. Zusammensetzung nach einem der Ansprüche 1 oder 2, **dadurch gekennzeichnet, dass** es sich bei den für R₁ und R₂ angegebenen Mono- oder Polyhydroxyalkylgruppen mit 2 bis 6 Kohlenstoffatomen um die Gruppen β-Hydroxyethyl und β,γ-Dihydroxypropyl handelt.

4. Zusammensetzung nach einem der Ansprüche 1 bis 3, **dadurch gekennzeichnet, dass** die Verbindungen der Formel (I) unter N,N-Bis(β-hydroxyethyl)amino-p-phenylendiamin,1-Amino-4-(N-β-hydroxyethyl-N-β-aminoethyl)amino-benzol, N,N'-Bis(β-hydroxyethyl)-N,N'-bis(4'-aminophenyl)-1,3-diamino-2-propanol, sowie den kosmetisch akzeptablen Salzen dieser Verbindungen ausgewählt sind.

5. Zusammensetzung nach einem der Ansprüche 1 bis 4, **dadurch gekennzeichnet, dass** das Oxidationsmittel unter Wasserstoffperoxid, Harnstoffperoxid, Alkalibromaten und Salzen von Persäuren, wie Perboraten und Persulfaten, ausgewählt ist.

6. Zusammensetzung nach einem der Ansprüche 1 bis 4, **dadurch gekennzeichnet, dass** sie einen pH-Wert im Bereich von 3 bis unter 7 aufweist.

7. Zusammensetzung nach einem der Ansprüche 1 bis 6, **dadurch gekennzeichnet, dass** der pH-Wert der Zusammensetzung (A) im Bereich von 3 bis 11 liegt.

8. Zusammensetzung nach einem der Ansprüche 1 bis 7, **dadurch gekennzeichnet, dass** der pH-Wert der Zusammensetzung (B) einen Wert von unter 7 aufweist.

9. Zusammensetzung nach einem der Ansprüche 1 bis 8, **dadurch gekennzeichnet, dass** das Farbstoffvorprodukt einer Oxidationsfarbe der Formel (I) vorzugsweise in Anteilen von 0,01 bis 4 Gew.-%, bezogen auf das Gesamtgewicht der resultierenden Zusammensetzung, vorliegt.

10. Zusammensetzung nach einem der Ansprüche 1 bis 9, **dadurch gekennzeichnet, dass** das m-Aminophenol und/oder 2-Methyl-5-aminophenol und/oder m-Dihydroxybenzol und/oder 2-Methyl-1,3-dihydroxybenzol und/oder ihre Salze in Anteilen von 0,005 bis 5 Gew.%, bezogen auf das Gesamtgewicht der resultierenden Zusammensetzung, vorliegen.

11. Zusammensetzung nach einem der Ansprüche 1 bis 10, **dadurch gekennzeichnet, dass** die Zusammensetzung (A) als zweiten Bestandteil außer den Farbstoffvorprodukten der Oxidationsfarben der Formel (I) weitere Farbstoffvorprodukte von Oxidationsfarben vom Typ der para- oder ortho-Verbindungen und/oder weitere Kuppler, die von den in Anspruch 1 definierten Verbindungen verschieden sind, und/oder Direktfarbstoffe enthält.

12. Zusammensetzung nach einem der Ansprüche 1 bis 11, **dadurch gekennzeichnet, dass** das zum Färben geeignete Medium aus Wasser oder einem Gemisch aus Wasser und einem Lösungsmittel besteht, das unter niederen C₁₋₄-Alkanolen, Glycerin, Glycolen, Glycolethern, aromatischen Alkoholen oder deren Gemischen ausgewählt ist.

13. Zusammensetzung nach einem der Ansprüche 1 bis 12, **dadurch gekennzeichnet, dass** sie ferner anionische, kationische, nichtionische, amphotere, zwitterionische grenzflächenaktive Stoffe oder ihre Gemische, Verdickungsmittel, Antioxidantien und/oder beliebige weitere kosmetisch akzeptable Hilfsstoffe enthält.

14. Verfahren zum oxidativen Färben von Keratinfasern und insbesondere menschlichen Keratinfasern, wie Haaren, **dadurch gekennzeichnet, dass** auf die Fasern eine Zusammensetzung aufgetragen wird, die durch Mischen unmittelbar vor der Verwendung einerseits einer Zusammensetzung (A), die in einem zum Färben geeigneten Medium enthalt:
· mindestens ein Farbstoffvorprodukt eines Oxidationsfarbstoffes der Formel (I): 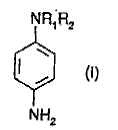 worin bedeuten:
R₁ Wasserstoff oder eine Mono- oder Polyhydroxyalkylgruppe mit 2 bis 6 Kohlenstoffatomen,
R₂ eine Mono- oder Polyhydroxyalkylgruppe mit 2 bis 6 Kohlenstoffatomen, β-Aminoethyl oder auch eine Gruppe der folgenden Formel (II): 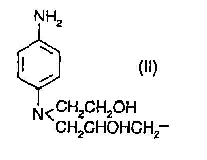 oder mindestens ein kosmetisch akzeptables Salz dieser Verbindungen,
· und mindestens einen Kuppler, der unter m-Aminophenol, 2-Methyl-5-aminophenol, m-Dihydroxybenzol, 2-Methyl-1,3-dihydroxybenzol und den kosmetisch akzeptablen Salzen dieser Verbindungen ausgewählt ist,
und andererseits einer Zusammensetzung (B) resultiert, die in einem zum Färben geeigneten Medium mindestens ein Oxidationsmittel enthält,
wobei der pH-Wert der Zusammensetzungen (A) und (B) so ist, dass nach dem Mischen der Zusammensetzung (A) mit der Zusammensetzung (B) in einem Gewichtsverhältnis von 0,5 bis 5 der pH-Wert der resultierenden Zusammensetzung unter 7 liegt, wobei der pH-Wert des bedarfsgemäß gebildeten Gemisches der Zusammensetzung (A) und (B) einer Wert unter 7 aufweist.

15. Verfahren nach Anspruch 14, **dadurch gekennzeichnet, dass** die Verbindungen der Formel (I) unter N,N-Bis(β-hydroxyethyl)-amino-p-phenylendiamin, 1-Amino-4-(N-β-hydroxyethyl-N-β-aminoethyl)amino-benzol, N,N'-Bis(β-hydrwcyethyl)-N,N'-bis(4'-aminophenyl)-1,3-diamino-2-propanol, sowie den kosmetisch akzeptablen Salzen dieser Verbindungen ausgewählt sind.

16. Verfahren nach Anspruch 14 oder 15, **dadurch gekennzeichnet, dass** das Oxidationsmittel unter Wasserstoffperoxid, Harnstoffperoxid, Alkalibromaten und Salzen von Persäuren, wie Perboraten und Persulfaten, ausgewählt ist.

17. Verfahren nach einem der Ansprüche 14 bis 16, **dadurch gekennzeichnet, dass** der pH-Wert der auf die Fasern aufgebrachten Zusammensetzung im Bereich von 3 bis unter 7 aufweist.

18. Verfahren nach einem der Ansprüche 14 bis 17, **dadurch gekennzeichnet, dass** die Zusammensetzung mit einer Einwirkungszeit von unter 10 min aufgetragen wird.

19. Verfahren nach einem der Ansprüche 14 bis 18, **dadurch gekennzeichnet, dass** die Zusammensetzung mit einer Einwirkungszeit von 3 bis 5 min aufgetragen wird.
